# EUROPEAN PATENT APPLICATION

(11) **EP 4 528 660 A1**
(43) Date of publication of application: **26.03.2025**
(21) Application number: 23910343.5
(22) Date of filing: 21.12.2023
(51) Int. Cl.: G06T 19/00

(54) **HUMAN-MACHINE INTERACTION COLLECTION METHOD, APPARATUS AND SYSTEM FOR WEARABLE EXTENDED REALITY DEVICE**

(30) Priority: 30.12.2022 CN 202211742603
(71) Applicant: Kingfar International Inc., Haidian District Beijing 100085 (CN)
(72) Inventor: ZHAO, Qichao, Beijing 100085 (CN); WANG, Qingju, Beijing 100085 (CN); YANG, Ran, Beijing 100085 (CN)
(74) Representative: Range, Christopher William
(86) International application number: PCT/CN2023/140683
(87) International publication number: WO 2024/140417

(57) **Abstract**

Provided are a human-machine interaction collection method, apparatus, and system for a wearable extended reality device. Human body state information is obtained by analyzing human body perception data collected. A three-dimensional environment scene corresponding to environmental perception data collected is obtained by modeling the environmental perception data collected. The human body state information and the three-dimensional environment scene are displayed in an integrated manner using an extended reality device. In this way, an individual can simultaneously obtain his/her own human body state information, the three-dimensional environment scene of his/her position, and an extended reality scene, which allows the individual to stay up-to-date on his/her own state and changes in a surrounding environment. The human body state information and the three-dimensional environment scene that are obtained are transmitted to a cloud center, in such a manner that the human body state information and the three-dimensional environment scene of a partner are available at all times through data sharing, which facilitates a realization of human-machine collaboration.

## Description

### CROSS-REFERENCE TO RELATED APPLICATION

This application claims priority to Chinese patent application No. CN202211742603.X, titled "HUMAN-MACHINE INTERACTION COLLECTION METHOD, APPARATUS, AND SYSTEM FOR WEARABLE EXTENDED REALITY DEVICE" and filed with China National Intellectual Property Administration on December 30, 2022, the entire contents of which are incorporated herein by reference.

### FIELD

The present disclosure relates to the field of wearable extended reality device technologies, and more particularly, to a human-machine interaction collection method, apparatus, and system for a wearable extended reality device.

### BACKGROUND

With the development of the times, a method for and an efficiency of obtaining information often determine a success or a failure of a matter, especially in occasions where one needs to keep abreast of latest information changes and personal changes. In the related art, a wearable intelligent device, including a physiological parameter sensor and/or a behavior observation system such as an eye tracker, an electroencephalograph and a Heart Rate (HR)/respiratory monitor, can detect a state of a person relying on multi-modal data such as physiological state data, eye movement data, and electroencephalographic data of the person. Devices such as cameras and lidars can be used to perceive object or spatial information of an environment to obtain real-time information about an environment where the person is located.

In addition, existing Extended Reality (XR) technologies include Augmented Reality (AR) technology, Virtual Reality (VR) technology, and Mixed Reality (MR) technology. These technologies allow a user to obtain more information and interact with others, the environment, etc. based on reality, or to obtain information and interact with others, the environment, etc. in a process of combining the reality and a virtual space. Taking the AR technology as an example, some products using the AR technology adopt a wearable design. The user can see additional virtual information overlaid on a real world through a pair of wearable AR glasses and other products. For example, the user can see a scene where the virtual information is superimposed on the real world through the AR glasses. At present, methods and technologies that can digitally model the real world are available. Relevant technicians and researchers can digitize the real world through modeling, and others can browse scenes from around the world through digitized results.

The related art can meet needs of people in a single aspect, including digital modeling of a physical world, a real-time collection of data information such as a physiological state and an eye movement of a person, Head-Up Display (HUD) information, and the like. However, existing AR/VR devices are unable to perceive a human body and a real environment simultaneously or model the real environment, preventing the user from interacting in real time with an environmental model that is based on the real environment.

### SUMMARY

In view of this, embodiments of the present disclosure provide a human-machine interaction collection method, apparatus, and system for a wearable extended reality device, to eliminate or mitigate one or more defects existing in the related art.

In an aspect of the present disclosure, a human-machine interaction collection method for a wearable extended reality device is provided. The method includes: obtaining data synchronously collected, the data including a virtual reality base signal collected by an extended reality device, human body perception data collected by a human body parameter perception device, and environmental perception data collected by an environmental parameter perception device, and the human body perception data including at least one of eye movement data, a physiological state parameter, and electroencephalographic data; providing an extended reality scene based on the virtual reality base signal collected by the extended reality device, obtaining human body state information of an individual based on the human body perception data collected by the human body parameter perception device, performing three-dimensional modeling based on the environmental perception data collected by the environmental parameter perception device, and obtaining a three-dimensional environment scene of a position of the individual; displaying the extended reality scene, the human body state information, and the three-dimensional environment scene on a display unit of the extended reality device; and uploading the human body state information of the individual and the three-dimensional environment scene to a cloud server.

In some embodiments of the present disclosure, the method further includes: obtaining a movement intention of the individual based on the human body perception data collected by the human body parameter perception device, converting the human body perception data based on which the movement intention is obtained into a control instruction for a collaborative device, and transmitting the control instruction to the collaborative device, to interact with the collaborative device through a change of the human body perception data.

In some embodiments of the present disclosure, the method further includes: generating relevant evaluation information based on the human body perception data and the environmental perception data, the relevant evaluation information including three-dimensional environment scene evaluation information and/or individual behavior decision information.

In some embodiments of the present disclosure, the method further includes: receiving a team behavior recommendation generated by the cloud server based on team behavior decision information subsequent to generating, by the cloud server, the team behavior decision information based on physical state information of a plurality of individuals in a team and the environmental perception data.

In some embodiments of the present disclosure, the human body parameter perception device and the environmental parameter perception device are integrated in the extended reality device.

In some embodiments of the present disclosure, the human body perception data includes the eye movement data. Said obtaining the human body state information of the individual based on the human body perception data collected by the human body parameter perception device includes: assessing physical state information of the individual using a pupil diameter, a blink count, a blink frequency, and/or an eye saccade behavior in the eye movement data, the physical state information including fatigue and/or cognitive load state information; and/or assessing mental state information of the individual using a fixation point, a fixation duration, and a fixation trajectory of the individual in the eye movement data, the mental state information including an attention distribution and/or an attention state.

In some embodiments of the present disclosure, the human body perception data includes the electroencephalographic data. Said obtaining the human body state information of the individual based on the human body perception data collected by the human body parameter perception device includes: assessing a physical state, mental state information, and/or emotional state information of the individual based on the electroencephalographic data collected by the human body parameter perception device.

In some embodiments of the present disclosure, the human body perception data includes the physiological state parameter. Said obtaining the human body state information of the individual based on the human body perception data collected by the human body parameter perception device includes: assessing emotional state information of the individual based on the physiological state parameter collected by the human body parameter perception device.

In some embodiments of the present disclosure, said obtaining the human body state information of the individual based on the human body perception data collected by the human body parameter perception device includes: obtaining a training data set based on historically collected human body perception data and a human body state information label corresponding to the historically collected human body perception data to train a machine model; and inputting human body perception data currently collected into the trained machine model to output, via the trained machine model, human body state information corresponding to the human body perception data currently collected.

In some embodiments of the present disclosure, the environmental perception data includes positioning data and image data. The environmental parameter perception device includes a camera and a radar. Said performing the three-dimensional modeling based on the environmental perception data collected by the environmental parameter perception device includes performing digital three-dimensional modeling on environmental data using simultaneous localization and mapping technology or a three-dimensional reconstruction technology.

In some embodiments of the present disclosure, the method further includes: obtaining a training data set based on data historically collected to train the machine model, and inputting data currently collected into the trained machine model to output, via the trained machine model, relevant evaluation information.

In some embodiments of the present disclosure, the three-dimensional modeling is performed through edge computing.

In another aspect of the present disclosure, a human-machine interaction collection apparatus of a wearable extended reality device is provided. The apparatus includes: an extended reality device integrated with a human body parameter perception device and an environmental parameter perception device and configured to collect a virtual reality base signal; the human body parameter perception device configured to perceive and collect human body perception data; the environmental parameter perception device configured to collect environmental perception data; a data processing module configured to convert a virtual reality base signal into an extended reality scene corresponding to a real scene, convert the collected human body perception data into human body state information of an individual, and convert the collected environmental perception data into a three-dimensional environment scene of a position of the individual; a display module configured to display the extended reality scene, the human body state information, and the three-dimensional environment scene that are obtained by the data processing module; and a data transmission module configured to upload the human body state information of the individual and the three-dimensional environment scene of the position of the individual to a cloud server.

In yet another aspect of the present disclosure, a human-machine interaction collection system based on an extended reality device is provided. The human-machine interaction collection system includes a processor and a memory. The memory stores computer instructions. The processor is configured to execute the computer instructions stored in the memory. The processor, when executing the computer instructions, implements the above-described human-machine interaction collection method that is based on the extended reality device.

In still yet another aspect of the present disclosure, a computer-readable storage medium is provided. The computer-readable storage medium stores a computer program. The computer program, when executed by a processor, implements the above-described human-machine interaction collection method that is based on the extended reality device.

With the human-machine interaction collection method, apparatus, and system based on the extended reality device of the present disclosure, human body perception data of a wearer of the extended reality device, environmental perception data of a position of the wearer, and virtual reality base data are synchronously collected by the human body parameter perception device, the environmental parameter perception device, and the extended reality device, or by integrating the human body parameter perception device and the environmental parameter perception device in the extended reality device. The human body state information, the three-dimensional environment scene, and the extended reality scene that are obtained are synchronously presented on the display unit. The wearer of the extended reality device can simultaneously obtain his/her own human body state information, three-dimensional environment scene of his/her position, the extended reality scene, and a real environment, which allows the wearer to stay up-to-date on his/her own state and changes in a surrounding environment. In addition, the human body state information and the three-dimensional environment scene that are obtained are transmitted to a cloud center, in such a manner that the human body state information and the three-dimensional environment scene of a partner are available at all times through data sharing, which facilitates a realization of human-machine collaboration.

Additional advantages, objects, and features of the present disclosure will be explained at least in part in the following description, and will become apparent to those skilled in the art upon examination of the following description, or can be learned from practicing of the present disclosure. The objects and other advantages of the present disclosure can be achieved and obtained by means of structures specifically pointed out in the specification and the accompanying drawings.

It will be appreciated by those skilled in the art that the objects and the advantages that can be achieved by the present disclosure are not limited to the above specific description. The above and other objects that can be achieved by the present disclosure will be more clearly understood from the following detailed description.

### BRIEF DESCRIPTION OF THE DRAWINGS

The accompanying drawings described herein are intended to provide a further understanding of the present disclosure, which constitute a part, rather than a limitation, of the present disclosure. The components in the accompanying drawings are not drawn to scale, but are merely intended to illustrate the principles of the present disclosure. To facilitate the illustration and description of some parts of the present disclosure, corresponding parts in the accompanying drawings may be enlarged, i.e., may become larger relative to other components in an exemplary device actually manufactured in accordance with the present disclosure.
FIG. 1 is a flowchart of a human-machine interaction collection of an extended reality device according to an embodiment of the present disclosure.
FIG. 2 is a flowchart of a signal transmission of a human-machine interaction collection apparatus for an extended reality device according to an embodiment of the present disclosure.
FIG. 3 is a diagram of an information transmission interaction structure between a cloud server and an extended reality device according to an embodiment of the present disclosure.
FIG. 4 is a schematic structural block diagram of a human-machine interaction collection apparatus for a wearable extended reality device according to an embodiment of the present disclosure.

### DETAILED DESCRIPTION

In order to make the objects, technical solutions, and advantages of the present disclosure more clearly understood, the present disclosure is further described in detail below with reference to the embodiments and the accompanying drawings. Exemplary embodiments of the present disclosure and descriptions thereof are intended to explain the present disclosure, and do not constitute a limitation of the present disclosure.

It should be further noted that, in order to avoid obscuring the present disclosure due to unnecessary details, only structures and/or processing steps closely related to the solutions according to the present disclosure are illustrated in the accompanying drawings, while other details that are not closely related to the present disclosure are omitted.

It should be emphasized that the terms "comprise/contain", when used in this specification, specify the presence of features, elements, steps, or components, but do not preclude the presence or addition of one or more other features, elements, steps, or components.

It should also be noted that, unless otherwise specified, the term "connection" may be used herein to refer not only to a direct connection but also to an indirect connection through an intermediate.

Hereinafter, the embodiments of the present disclosure will be described with reference to the accompanying drawings. In the accompanying drawings, same or similar components or same or similar steps are denoted by same reference numerals.

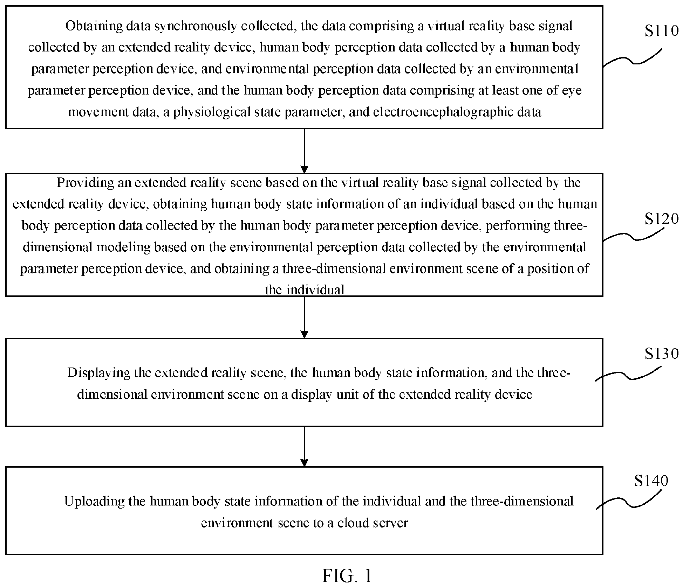

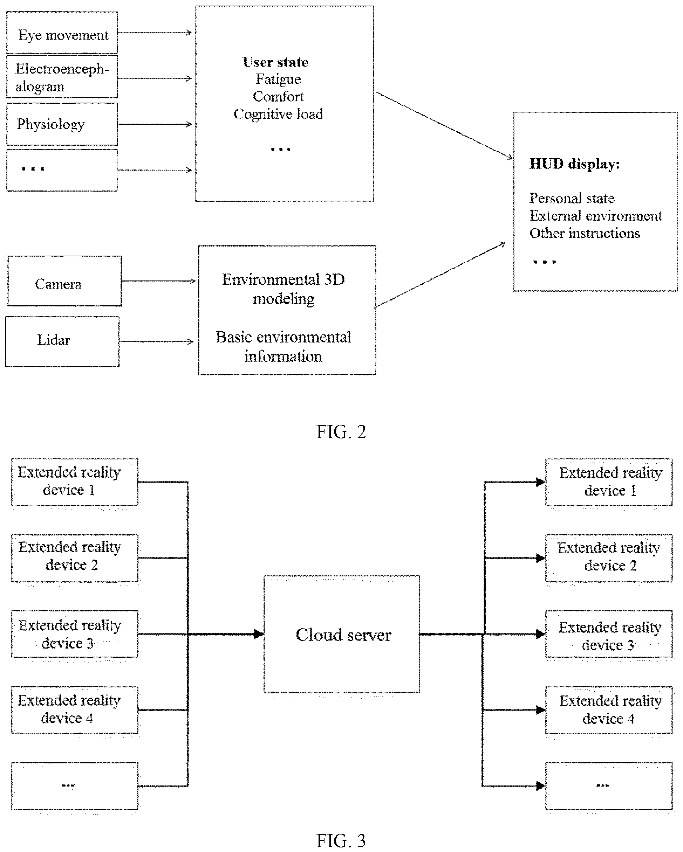

To enable a user to stay up-to-date on his/her own state and changes in a surrounding environment based on an environmental model of a real environment, and to perform a real-time interaction based on his/her state and the changes in the environment, a human-machine interaction collection method for an extended reality device is provided according to an embodiment of the present disclosure. As illustrated in FIG. 1, the method includes operations at blocks S110 to S140. A corresponding signal transmission flow is illustrated in FIG. 2.

At block S110, data synchronously collected is obtained. The data synchronously collected includes a virtual reality base signal collected by an extended reality device, human body perception data collected by a human body parameter perception device, and environmental perception data collected by an environmental parameter perception device.

The human body perception data includes at least one of eye movement data, a physiological state parameter, and electroencephalographic data. In the embodiments of the present disclosure, the physiological state parameter (or referred to as physiological state data) represents a parameter indicating a physiological state of a human body other than the eye movement data and the electroencephalographic data, and includes, but is not limited to, one or more of a heart rate (HR) indicator, an electrocardiogram indicator, a skin temperature (SKT) indicator, a electrodermal activity (EDA), a respiration indicator and/or a blood oxygen indicator, etc. However, the present disclosure is not limited to any of these examples.

In the above operation at block S110, based on Network Time Protocol (NTP) clock synchronization technology, the virtual reality base data, the human body perception data, and the environmental perception data are simultaneously collected by the extended reality device, the human body parameter perception device, and the environmental parameter perception device, respectively. The virtual reality base data, the human body perception data, and the environmental perception data are collected on a same timeline. In this way, the human body perception data of an individual over time, the virtual reality base data of a position of the individual, and the environmental parameter perception data are obtained in real time. In an embodiment of the present disclosure, the human body parameter perception device, and the environmental parameter perception device may be or may not be integrated into the extended reality device. The virtual reality base data may include information such as positions and/or appearances of various objects in the real environment collected by a collection device such as a camera and/or a radar. The human body perception data may include physiological signals such as the eye movement data, the physiological state data, and/or the electroencephalographic data collected by a human body physiological signal collection device such as an eye movement device, an electroencephalographic device, and/or a physiological state parameter sensor. The environmental perception data may include data such as a position, an appearance, a structure, and/or a dimension of a feature point in the real environment collected by an environmental collection device such as a camera and/or a radar.

In an embodiment, for a collection of the eye movement data, a pupil-corneal reflex method may be used to collect eye movements and change data such as a pupil diameter, a blink count, a blink frequency, and/or an eye saccade behavior of the individual. The eye movement data such as a fixation point, a fixation duration, and a fixation trajectory of the individual may also be collected. For a collection of the physiological state data, physiological signals such as HR, EDA, and SKT are collected through a collection of relevant electrical signals or through photoplethysmography technology. For a collection of the electroencephalographic data, a method using an electroencephalographic dry electrode is adopted to collect physiological signals such as an electroencephalographic spectrum. The environmental perception data includes positioning data and image data. The environmental parameter perception device includes a camera and a radar.

At block S120, an extended reality scene is provided based on the virtual reality base signal collected by the extended reality device, human body state information of an individual is obtained based on the human body perception data collected by the human body parameter perception device, three-dimensional modeling is performed based on the environmental perception data collected by the environmental parameter perception device, and a three-dimensional environment scene of a position of the individual is obtained.

In the above operation at block S 120, providing the extended reality scene based on the virtual reality base signal collected by the extended reality device includes forming an extend reality scene based on the virtual reality base signal collected by adding virtual extended information to the real environment as desired. For example, information such as an attention identifier and an extra item is added to the real environment. Based on the information such as the attention identifier and the extra item, the extended reality device can prompt dangerous information that may exist in a current environment, such as a hidden space that is difficult to be observed by naked eyes, creatures hidden in a space (information that can be obtained through thermal infrared technology, etc.), special terrain that has been identified, and other like information; and/or the extended reality device can prompt a current state of the user, such as an emotional stress level, a fatigue level, and a distraction level. When the current state of the user exceeds a predetermined value, feedback warning can be carried out, including vibration feedback, visual feedback, auditory feedback, etc.

Obtaining the human body state information of the individual based on the human body perception data collected by the human body parameter perception device includes: assessing physical state information such as fatigue and/or cognitive load state of the individual using the eye movement data collected of the individual such as the pupil diameter, the blink count, the blink frequency, and/or the eye saccade behavior; assessing mental state information such as an attention distribution and an attention state of the individual using the eye movement data collected of the individual such as the fixation point, the fixation duration, and the fixation trajectory; assessing an emotion state such as excitement, calmness, tension, and agitation of the individual using changes in indicators such as Standard Deviation of NN intervals (SDNN) and LF/HF (i.e., a sympathetic and parasympathetic nerve dynamic balance indicator) of Heart Rate Variability (HRV) collected, and changes in indicators such as Skin Conductance (SC) of EDA; and assessing the physical state information, the mental state information, the emotional state information, and other information such as fatigue, excitement, calmness, anger, and/or cognitive load of the individual by performing a frequency domain analysis, a time domain analysis, a Power Spectral Density (PSD) electroencephalographic signal power spectrum analysis, or other analyses on an electroencephalographic signal collected.

The physiological signal is related to peripheral autonomic nerve activities of the human body, and can reflect the changes in the physical state, the mental state, the emotional state, or the like of a person. The operation of obtaining the human body state information of the individual based on the human body perception data collected by the human body parameter perception device includes: obtaining a training data set based on historically collected human body perception data and a corresponding human body state information label to train a machine model; and inputting human body perception data currently collected into the trained machine model to output, via the trained machine model, human body state information corresponding to the human body perception data currently collected. The machine model is configured to obtain the training data set based on historically collected data in various types of human body perception data collected by the wearable extended reality device and a corresponding human body state information label. The trained machine model obtained is configured to use the human body perception data collected in real time by the wearable extended reality device as input values, and correspondingly output human body state information at each time point. An operation of obtaining the human body state information of the individual based on the human body perception parameter including the eye movement data, the physiological state parameter, and/or the electroencephalographic data includes: collecting the human body perception data required for identifying the human body state information, such as the eye movement data, the physiological state parameter, and/or the electroencephalographic data; and preprocessing each type of human body perception data collected to facilitate a feature extraction and performing a further feature data extraction. Each of historical human body perception data collected and corresponding human body state information can be used as labels of training samples to generate the training data set. Based on the various types of human body perception data in the training data set and corresponding labels, a corresponding machine learning model can be trained using extracted feature data. Different machine models can be trained based on the corresponding human body state information and different types of physiological signals such as the eye movement data, the electroencephalographic data, and the physiological state parameter. Then, various types of human body perception data currently collected are input into the corresponding trained machine learning model for a prediction to obtain various types of current human body state information of the individual. For example, when the person is in a state of fatigue, tension, stress, or excitement, an HRV signal of the person changes greatly compared with a resting state. An operation of obtaining the human body state information of the individual based on the HRV signal includes: collecting a Photo Plethysmo Graphy (PPG) (pulse) signal of the individual; obtaining the HRV signal by performing a calculation processing on the collected PPG signal; denoising the HRV signal using wavelet noise reduction, high/low pass filtering, band-stop filtering, ectopic interval detection or ectopic interval correction, or other methods; analyzing the HRV signal through methods such as a time domain analysis, a frequency domain analysis, and/or a nonlinear analysis; extracting an HRV time domain feature value, an HRV frequency domain feature value, and/or an HRV nonlinear feature value; and inputting the extracted feature values into Support Vector Machine (SVM), random forest, and other algorithm models to classify data, for obtaining corresponding human body state information of the individual, such as a stress level (low, medium, high).

In another embodiment of the present disclosure, the wearable extended reality device may be a pair of wearable extended reality glasses. A plurality of human body parameter perception devices may be integrated into the pair of wearable extended reality glasses to monitor changes in the human body perception data of the user in real time. The human body state information of the wearer is assessed through a built-in algorithm and a threshold standard. An assessment result of the human body state information is displayed on the glasses in real time. In addition, the user is allowed to assess and change his/her action based on his/her actual situation. For example, a fatigue state, a tense state, a calm state, an angry state, or the like of the wearer is displayed on the glasses.

Performing the three-dimensional modeling based on the environmental perception data collected by the environmental parameter perception device to obtain the three-dimensional environment scene of the position of the individual includes performing digital three-dimensional modeling on environmental data using a Simultaneous Localization and Mapping (SLAM) technology or a three-dimensional reconstruction technology to obtain the three-dimensional environment scene. The three-dimensional modeling is performed through edge computing, which ensures that a slow data transmission, data transmission congestion, or the like is unlikely to occur due to an excessive data quantity.

In some embodiments of the present disclosure, the operation at block S120 may further include generating relevant evaluation information based on the human body perception data and the environmental perception data. The relevant evaluation information includes three-dimensional environment scene evaluation information and/or individual behavior decision information.

The above-described operation of generating the relevant evaluation information based on the human body perception data and the environmental perception data includes: obtaining a training data set based on data historically collected (the human body perception data and the environmental perception data) and the generated relevant evaluation information (the three-dimensional environment scene evaluation information and/or the individual behavior decision information, which are used as labels) to train the machine model, and inputting data currently collected into the trained machine model to output, via the trained machine model, relevant evaluation information corresponding to the data currently collected. The three-dimensional environment scene evaluation information and the individual behavior decision information may correspond to different trained machine models. Each machine model is configured to obtain the training data set based on the human body perception data collected by the wearable extended reality device, historically collected data of the environmental perception data, and the corresponding relevant evaluation information (which is used as the labels). The obtained machine model is configured to take the human body perception data collected by the wearable extended reality device in real time and the environmental perception data as input values, and correspondingly output relevant evaluation information at each time point. The relevant evaluation information includes the three-dimensional environment scene evaluation information or the individual behavior decision information. The environment scene evaluation information may include, for example, information such as complexity of current topography, whether or not a potential danger exists in the current environment, and temperature and humidity of the current environment. The individual behavior decision information may include, for example, information such as individual decision-making preference and individual behavior performance.

In some embodiments of the present disclosure, in a case where the human body perception data includes the eye movement data, the relevant evaluation information is the three-dimensional environment scene evaluation information. The operation of generating the relevant evaluation information based on the human body perception data and the environmental perception data includes: determining a fixation point of an eye in the three-dimensional environment scene based on the eye movement data and the environmental perception data, and generating, based on the determined fixation point, the three-dimensional environment scene evaluation information in accordance with a predetermined evaluation strategy. For example, the eye movement data of the fixation point of the individual in the environment and the environmental perception data of the position of the individual are simultaneously collected by the extended reality device, and then the three-dimensional environment scene of the position of the individual and a fixation change trajectory of the eye in the three-dimensional environment scene are simultaneously obtained to form a three-dimensional fixation point of the individual. Whether a change of a concern point of the individual in the environment achieves an effect desired by an environment designer is analyzed using the three-dimensional fixation point of the individual, in such a manner that a design of a real scene is evaluated and analyzed.

In other embodiments of the present disclosure, in a case where the human body perception data includes the physiological state parameter (for example, the HR indicator, the electrocardiogram indicator, EDA, and/or SKT), the operation of generating the relevant evaluation information based on the human body perception data and the environmental perception data includes: generating, by a cloud center server or the extended reality device, the individual behavior decision information based on the individual human body state information of the individual and the environmental perception data; generating an individual behavior recommendation based on the individual behavior decision information; and outputting, by the extended reality device, the individual behavior recommendation. For example, the physiological state parameter of the individual in the environment and the environmental perception data of the position of the individual are simultaneously collected by the extended reality device, and then the human body state information of the individual and the three-dimensional environment scene of the position of the individual are simultaneously obtained to assist the user in determining his/her own situation and environmental conditions, in such a manner that more rational decisions can be made. For example, physical energy consumption of the user is displayed to assist the user in determining his/her action ability, in such a manner that the user can decide his/her action range and rest time more rationally.

In other embodiments of the present disclosure, in a case where the human body perception data includes the electroencephalographic data, generating the relevant evaluation information based on the human body perception data and the environmental perception data includes: generating, by the cloud center server or the extended reality device, the individual behavior decision information based on the human body state information and the environmental perception data; generating the individual behavior recommendation based on the individual behavior decision information; and outputting, by the extended reality device, the individual behavior recommendation. For example, the electroencephalographic data of the individual and the environmental perception data of the position of the individual are simultaneously collected by the extended reality device, and then the human body state information of the individual and the three-dimensional environment scene of the position of the individual are simultaneously obtained, to determine whether the individual can perform a corresponding human-machine interaction task under a current condition of the electroencephalographic data, and determine whether a task execution device should take on a current task. For example, when state information of the individual obtained based on the electroencephalographic data shows a low level of fatigue, the task execution device is determined to take on the current task; and when the state information of the individual obtained based on the electroencephalographic data shows a high level of fatigue, the task execution device is determined not to take on the current task.

In some embodiments of the present disclosure, the above-described operation at block S120 may further include: obtaining a movement intention of the individual based on the human body perception data collected by the human body parameter perception device, and converting the human body perception data based on which the movement intention is obtained into a control instruction for a collaborative device; and interacting with the collaborative device through a change of the human body perception data.

The human body perception data such as the eye movement data, the physiological state parameter, and/or the electroencephalographic data that are collected by the human body parameter perception device may be converted into an instruction signal for a specific collaborative device in combination with a predetermined artificial intelligence algorithm in a data processing and analysis module. In this way, communication with the specific collaborative device is completed. By interacting with the specific collaborative device based on a change in multi-modal human body perception data such as an eye movement behavior, a physiological state change, and an electroencephalographic change, a human-machine collaborative operation between the individual and a machine system such as a drone and a collaborative operation between individuals can be achieved.

In some embodiments of the present disclosure, the operation of converting the human body perception data into the instruction signal for the specific collaborative device includes: collecting the human body perception data required for generating the instruction signal; preprocessing the human body perception data to facilitate a feature extraction, and performing a further feature extraction; classifying extracted feature data, in such a manner that different types of classified feature data point to different movement intentions for the collaborative device; and converting each type of classified feature data into the control instruction for the collaborative device. For example, when a person imagines his/her limb movements or muscle movements without actually outputting any movement, a specific brain region of the person is still activated. By analyzing the electroencephalographic signal, activation effects of different brain regions are detected and identified to determine an intention of the user, in such a manner that direct communication and control between a human brain and an external device is realized. The operation of converting the electroencephalographic signal into the control instruction for the collaborative device includes: collecting the electroencephalographic data through adopting the method using the electroencephalographic dry electrode; processing the collected electroencephalographic data using an algorithm such as power spectrum analysis, wavelet transform, Autoregressive model (AR), Sample Entropy (SampEn) or Common Spatial Pattern (CSP) and extracting the feature data; classifying the extracted feature data using an algorithm such as Linear Discriminant Analysis (LDA), Support Vector Machines (SVM), Artificial Neural Network (ANN) or Bayes Classifier to obtain a variety of types of classified feature data; converting, by a brain-computer interaction system based on Motor Imagery (MI), a brain-computer interaction system based on Steady-State Visual Evoked Potential (SSVEP), or a brain-computer interaction system based on P300, the classified feature data into the control instruction for the collaborative device, in such a manner that the human-machine interaction is completed.

In some embodiments of the present disclosure, the wearable extended reality device is configured to collect the physiological state parameter of the individual. By analyzing and processing changes in a physiological state of the user, an interaction between the user and an auxiliary operation device is realized to complete a collaborative operation. For example, by collecting and analyzing a heart rate change of the individual, a current emotional state of the individual is identified. Whether the auxiliary operation device needs to take on a current task is determined by determining whether the individual is currently suitable for performing the current task.

In another embodiment, the wearable extended reality device is configured to collect the eye movement data of the individual. By analyzing and processing changes in the eye movement data, communication with a specific collaborative device is carried out to realize a human-machine collaborative operation between the user and the auxiliary operation device. For example, an eye fixation duration and fixation position data are identified to perform a relevant operation on a position of the fixation point.

In another embodiment, the wearable extended reality device is configured to collect the electroencephalographic data of the individual. By analyzing and processing changes in brain waves, the interaction between the user and the auxiliary operation device is realized to complete the collaborative operation. For example, by collecting and analyzing the changes in the brain waves of the user, the auxiliary operation device can execute a corresponding task based on electroencephalographic information of the user, including controlling a direction of an operation device, a state of executing the task, etc.

At block S130, the extended reality scene, the human body state information, and the three-dimensional environment scene are displayed on a display unit of the extended reality device.

The display unit of the extended reality device is made of a light-transmitting picture presentation medium. The wearer of the extended reality device can observe the real environment through the display unit and freely move in a specific place. One or more of the extended reality scene, the human body state information, and the three-dimensional environment scene are selectively presented on the display unit. The extended reality scene and the three-dimensional environment scene overlap based on positions and fixed forms of corresponding identifier points in the extended reality scene and the three-dimensional environment scene. Based on a position and a fixed form of an identifier point in the real environment, a display picture on the display unit overlaps with the real environment. The wearer of the extended reality device can see one or more of the extended reality scene, the three-dimensional environment scene, and the real scene simultaneously, which helps the wearer to understand his/her surrounding environment more clearly and deeply. Presenting the human body state information on the display unit helps the wearer to understand his/her own state more clearly, in such a manner that the wearer can adjust his/her own movement, spirit, emotion, and other states in a timely and reasonable manner, to adjust his/her own action plan. For example, a personnel state recognition such as a recognition of fatigue, high load, excitement, anger, or the like is performed based on the collected multi-modal data of the wearer of the extended reality device. A state recognition result is reflected in the extended reality device in real time, including imaging display of the display unit, a voice reminder, a vibration reminder, etc.

In the above operation at block S 130, the display unit of the extended reality device may be further configured to display human body state information and three-dimensional environment scene from another extended reality device of a cloud server.

At block S140, the human body state information of the individual and the three-dimensional environment scene are uploaded to the cloud server.

The human body state information of the wearer of the extended reality device and the three-dimensional environment scene are uploaded to the cloud server, in such a manner that all collaborative operators and commanders can obtain the human body state information and the three-dimensional environment scene of others through the cloud server. In this way, work such as collaborative sharing of team messages, a real-time update of strategies, and an adjustment of division of labor among team members is facilitated. Further, a remote human-machine interaction can also be achieved by remotely controlling a device through the human body perception data. For an application scenario with many operators, an information transmission interaction structure between the cloud server and each extended reality device is illustrated in FIG. 3.

In some embodiments of the present disclosure, each of extended reality devices 1, 2, 3, 4, etc. can be configured to obtain three-dimensional data coordinates of a physical world of a fixation position of the wearer in real time, digitally model the physical world in real time, and upload the digitally modeled physical world to the cloud server in real time, in such a manner that remote commanders and other people using the devices can share such geographic information.

In some embodiments of the present disclosure, the above operation at block S140 may further include: generating, by the cloud server, team behavior decision information based on the physical state information of a plurality of individuals in a team and the environmental perception data; and generating, by the cloud server, a team behavior recommendation based on the team behavior decision information. The team behavior recommendations may be transmitted to extended reality devices 1, 2, 3, 4, etc.

In some embodiments of the present disclosure, the human body state data of the user of the wearable extended reality device is obtained and transmitted to a team leader of a team to which the user belongs. In this way, the team leader can grasp physical conditions of team members in real time, which assists the team leader in making more scientific and rational decisions. For example, the team leader may grasp physical energy consumption of all the team members in real time. If the physical energy consumption of most team members has reached 80%, the team leader tells the team to stop moving and find a place to rest.

With the human-machine interaction collection method based on the extended reality device of the present disclosure, the human body perception data of the wearer of the extended reality device, the environmental perception data of the position of the wearer, and the virtual reality base data are synchronously collected by integrating the human body parameter perception device and the environmental parameter perception device in the extended reality device. The human body state information, the three-dimensional environment scene, and the extended reality scene that are obtained are synchronously presented on the display unit. The wearer of the extended reality device can simultaneously obtain his/her own human body state information, three-dimensional environment scene of his/her position, the extended reality scene, and the real environment, which allows the wearer to stay up-to-date on his/her own state and changes in the surrounding environment. In addition, the human body state information and the three-dimensional environment scene that are obtained are transmitted to the cloud center, in such a manner that the human body state information and the three-dimensional environment scene of a partner are available at all times through data sharing, which facilitates a realization of human-machine collaboration.

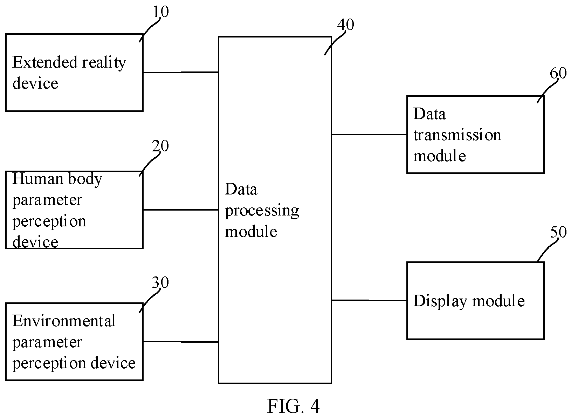

Corresponding to the above-described method, the present disclosure further provides a human-machine interaction collection apparatus for a wearable extended reality device. The human-machine interaction collection apparatus is an integrated apparatus, preferably a glasses-type apparatus, but the present disclosure is not limited thereto. As illustrated in FIG. 4, the human-machine interaction apparatus includes an extended reality device 10, a human body parameter perception device 20, an environmental parameter perception device 30, a data processing module 40, a display module 50, and a data transmission module 60. The extended reality device 10 is configured to collect a virtual reality base signal. The human body parameter perception device 20 is configured to perceive and collect human body perception data. The environmental parameter perception device 30 is configured to collect environmental perception data. The human body perception data includes at least one of eye movement data, physiological state parameter, and electroencephalographic data. An eye tracker (including an infrared light source and a camera) required for collecting the eye movement data, a light source and an electrode required for collecting the physiological state parameter, an electroencephalographic device (including an electroencephalographic dry electrode) for collecting the electroencephalographic data, and a camera and a radar for collecting the environmental perception data can all be integrated into the wearable extended reality device. A data collection is free from being restricted by the environment and conditions. In an embodiment of the present disclosure, the human body parameter perception device, the environmental parameter perception device, and the extended reality device may be separately provided, or the human body parameter perception device and the environmental parameter perception device may be integrated into the extended reality device.

The data processing module 40 is configured to convert a virtual reality base signal into an extended reality scene corresponding to a real scene, convert the collected human body perception data into human body state information of an individual, and convert the collected environmental perception data into a three-dimensional environment scene of a position of the individual. The data processing module 40 is further configured to convert the collected human body perception data into a control instruction for a collaborative device, and generate relevant evaluation information based on the human body perception data and the environmental perception data. The relevant evaluation information includes three-dimensional environment scene evaluation information and/or individual behavior decision information.

The display module 50 is configured to display the extended reality scene, the human body state information, and the three-dimensional environment scene that are obtained by the data processing module. The display module 50 is further configured to display the human body state information and the three-dimensional environment scene from another extended reality device of a cloud server.

The data transmission module 60 is configured to upload the human body state information of the individual and the three-dimensional environment scene of the position of the individual to the cloud server.

Corresponding to the above-described method, the present disclosure further provides a human-machine interaction collection system for a wearable extended reality device. The system includes a computer device. The computer device includes a processor and a memory. The memory stores computer instructions. The processor is configured to execute the computer instructions stored in the memory. The processor, when executing the computer instructions, implements the above-described human-machine interaction collection method for the wearable extended reality device.

According to an embodiment of the present disclosure, a computer-readable storage medium is further provided. The computer-readable storage medium stores a computer program. The computer program, when executed by a processor, implements the above-described human-machine interaction collection method for the wearable extended reality device. The computer-readable storage medium may be a tangible storage medium such as a Random Access Memory (RAM), a memory, a Read-Only Memory (ROM), an electrically programmable ROM, an electrically erasable programmable ROM, a register, a floppy disk, a hard disk, a removable storage disk, a CD-ROM, or any other form of storage medium known in the art.

Those skilled in the art could be aware that, exemplary components, systems, and methods described in combination with embodiments disclosed herein may be implemented by hardware, software, or a combination thereof. Whether these functions are executed by hardware or software is dependent on particular use and design constraints of the technical solutions. Professionals may adopt different methods for different particular uses to implement described functions, which should not be regarded as going beyond the scope of the present disclosure. When implemented in hardware, elements of the present disclosure may be, for example, electronic circuits, Application Specific Integrated Circuits (ASICs), appropriate firmware, plug-ins, and function cards. When implemented in software, the elements of the present disclosure are programs or code segments used to execute required tasks. The programs or the code segments may be stored in a machine-readable medium or transmitted over a transmission medium or a communication link via a data signal carried in a carrier wave.

It should be understood that the present disclosure is not limited to specific configurations and processings described above and illustrated in the figures. For the sake of conciseness, a detailed description of known methods is omitted here. In the above-described embodiments, several specific steps are described and illustrated as examples. However, the process of the method of the present disclosure is not limited to the specific steps described and illustrated. Those skilled in the art can make various changes, modifications, and additions, or change the order of the steps after grasping the spirit of the present disclosure.

In the present disclosure, features described and/or illustrated for one embodiment may be used in the same manner or in a similar manner in one or more other embodiments, and/or may be combined with or substituted for features of other embodiments.

Although some embodiments of the present disclosure are described above, the present disclosure is not limited to these embodiments. For those skilled in the art, various changes and variations can be made to the embodiments of the present disclosure. Any modification, equivalent replacement, improvement, etc. made within the spirit and principle of the present disclosure shall fall within the protection scope of the present disclosure.

## Claims

1. A human-machine interaction collection method for a wearable extended reality device, the method comprising:
obtaining data synchronously collected, the data comprising a virtual reality base signal collected by the extended reality device, human body perception data collected by a human body parameter perception device, and environmental perception data collected by an environmental parameter perception device, and the human body perception data comprising at least one of eye movement data, a physiological state parameter, and electroencephalographic data;
providing an extended reality scene based on the virtual reality base signal collected by the extended reality device, obtaining human body state information of an individual based on the human body perception data collected by the human body parameter perception device, performing three-dimensional modeling based on the environmental perception data collected by the environmental parameter perception device, and obtaining a three-dimensional environment scene of a position of the individual;
displaying the extended reality scene, the human body state information, and the three-dimensional environment scene on a display unit of the extended reality device; and
uploading the human body state information of the individual and the three-dimensional environment scene to a cloud server.

2. The method according to claim 1, further comprising:
obtaining a movement intention of the individual based on the human body perception data collected by the human body parameter perception device, converting the human body perception data based on which the movement intention is obtained into a control instruction for a collaborative device, and transmitting the control instruction to the collaborative device, to allow an interaction with the collaborative device through a change of the human body perception data.

3. The method according to claim 1, further comprising:
generating relevant evaluation information based on the human body perception data and the environmental perception data, the relevant evaluation information comprising three-dimensional environment scene evaluation information and/or individual behavior decision information.

4. The method according to claim 3, further comprising:
receiving a team behavior recommendation generated by the cloud server based on team behavior decision information subsequent to generating, by the cloud server, the team behavior decision information based on physical state information of a plurality of individuals in a team and the environmental perception data.

5. The method according to claim 1, wherein the human body parameter perception device and the environmental parameter perception device are integrated in the extended reality device.

6. The method according to claim 1, wherein the human body perception data comprises the eye movement data, and
said obtaining the human body state information of the individual based on the human body perception data collected by the human body parameter perception device comprises:
assessing physical state information of the individual using a pupil diameter, a blink count, a blink frequency, and/or an eye saccade behavior in the eye movement data, the physical state information comprising fatigue and/or cognitive load state information; and/or
assessing mental state information of the individual using a fixation point, a fixation duration, and a fixation trajectory of the individual in the eye movement data, the mental state information comprising an attention distribution and/or an attention state.

7. The method according to claim 1, wherein the human body perception data comprises the electroencephalographic data, and
said obtaining the human body state information of the individual based on the human body perception data collected by the human body parameter perception device comprises:
assessing physical state information, mental state information, and/or emotional state information of the individual based on the electroencephalographic data collected by the human body parameter perception device.

8. The method according to claim 1, wherein the human body perception data comprises the physiological state parameter, and
said obtaining the human body state information of the individual based on the human body perception data collected by the human body parameter perception device comprises:
assessing emotional state information of the individual based on the physiological state parameter collected by the human body parameter perception device.

9. The method according to claim 1, wherein said obtaining the human body state information of the individual based on the human body perception data collected by the human body parameter perception device comprises:
obtaining a training data set based on human body perception data historically collected and a corresponding human body state information label, to train a machine model; and
inputting human body perception data currently collected into the trained machine model to output, by the trained machine model, human body state information corresponding to the human body perception data currently collected.

10. The method according to claim 1, wherein:
the environmental perception data comprises positioning data and image data;
the environmental parameter perception device comprises a camera and a radar; and
said performing the three-dimensional modeling based on the environmental perception data collected by the environmental parameter perception device comprises performing digital three-dimensional modeling on environmental data using a simultaneous localization and mapping technology or a three-dimensional reconstruction technology.

11. The method according to claim 3, wherein said generating the relevant evaluation information based on the human body perception data and the environmental perception data comprises:
generating a training data set based on human body perception data historically collected, the environmental perception data historically collected, and corresponding relevant evaluation information, to train a machine model; and
inputting human body perception data and the environmental perception data that are currently collected into the trained machine model to output, by the trained machine model, relevant evaluation information corresponding to data currently collected.

12. The method according to claim 1, wherein the three-dimensional modeling is performed through edge computing.

13. The method according to claim 1, wherein the wearable extended reality device is a pair of wearable extended reality glasses.

14. A human-machine interaction collection apparatus for a wearable extended reality device, the human-machine interaction collection apparatus comprising:
an extended reality device configured to collect a virtual reality base signal;
a human body parameter perception device configured to perceive and collect human body perception data, the human body perception data comprising at least one of eye movement data, a physiological state parameter, and electroencephalographic data;
an environmental parameter perception device configured to collect environmental perception data;
a data processing module configured to convert the virtual reality base signal into an extended reality scene corresponding to a real scene, convert the collected human body perception data into human body state information of an individual, and convert the collected environmental perception data into a three-dimensional environment scene of a position of the individual;
a display module configured to display the extended reality scene, the human body state information, and the three-dimensional environment scene that are obtained from the data processing module; and
a data transmission module configured to upload the human body state information of the individual and the three-dimensional environment scene of the position of the individual to a cloud server.

15. A human-machine interaction collection system for a wearable extended reality device, the human-machine interaction collection system comprising:
a processor; and
a memory having computer instructions stored thereon, wherein the processor is configured to execute the computer instructions stored in the memory, the processor, when executing the computer instructions, implementing the method according to any one of claims 1 to 13.

16. A computer-readable storage medium having a computer program stored thereon, wherein the computer program, when executed by a processor, implements the method according to any one of claims 1 to 13.
